# EUROPEAN PATENT APPLICATION

(11) **EP 4 763 217 A1**
(43) Date of publication of application: **24.06.2026**
(21) Application number: 24220746.2
(22) Date of filing: 17.12.2024
(51) Int. Cl.: A61K 38/18, A61K 9/00, A61P 25/00, A61P 27/00, A61P 27/02

(54) **INTRANASAL ADMINISTRATION OF NGF FOR THE PREVENTION OR TREATMENT OF RETINOPATHIES AND OPTIC NERVE DISORDERS**

(71) Applicant: Dompé farmaceutici SpA, 20122 Milano (IT)
(72) Inventor: ALLEGRETTI, Marcello, 67100 L'Aquila (IT); CATTANI, Franca, 67100 L'Aquila (IT); ANTONANGELI, Maria Irene, 67100 L'AQUILA (IT)
(74) Representative: Dompé farmaceutici Spa

(57) **Abstract**

The present invention relates to Nerve growth factor (NGF) for use in the prevention or treatment of a retinopathy or of an optic nerve disease in a subject, wherein said NGF is administered intranasally to the subject.

## Description

### FIELD OF THE INVENTION

The present invention relates to NGF for use in the prevention or treatment of a retinopathy or of an optic nerve disorder in a subject, wherein said NGF is administered intranasally to the subject.

### STATE OF THE ART

The nerve growth factor (NGF) is a member of the family of evolutionarily well-conserved neurotrophin growth factors that are required for the development and survival of specific neuronal populations, which also includes brain-derived neurotrophic factor (BDNF), neurotrophin-3 (NT3) and NT4/5. The NGF sequence is well preserved among different species, with 90% homology between murine and human NGF.

NGF and its receptors are expressed both in the retina and in the optic nerve. Specifically, NGF is expressed by retinal pigment epithelium, Müller cells, and retinal ganglion cells. NGF receptors are found on retinal pigment epithelium, photoreceptors, Müller cells, retinal ganglion cells, in the visual cortex and in oligodendrocytes of the optic nerve (Carmignoto G, etal., Exp Neurol. 1991;111(3):302-311; Chakrabarti S, et al., Brain Res. 1990;523(1):11-15; Lambiase A, et al., Invest Ophthalmol Vis Sci. 1998;39:1272-1275; Rossi FM et al., J Neurosci. 2002;22(3):912-919; Cohen RI et al., J Neurosci. 1996;16(20):6433-6442).

There is both preclinical and clinical evidence in the literature of a therapeutic potential of NGF for the treatment of retinopathies and optic nerve disorders (Kanu et al., Semin Ophthalmol. 2021 May 19; 36(4): 224-231).

Preclinical studies demonstrated that the administration of NGF is effective in several pathological settings involving the retina and the optic nerve, including optic nerve transection (Carmignoto G. et al., J Neurosci Off J Soc Neurosci. 1989;9(4):1263-1272), retinal detachment (Xiaodong Sun et al., Ophthalmologica 2008;222(1):58-61), phototoxic retinopathy (Rocco et al., Graefes Arch Clin Exp Ophthalmol 2018, 256: 729-738), retinal ischemia (Siliprandi R. et al., Invest Ophthalmol Vis Sci. 1993; 34:3232-3245; Chen et al., Growth Factors 2015;33(2):149-159), glaucoma (Colafrancesco V. et al., J Glaucoma. 2011; 20(2):100-108; Lambiase A. et al., Proc Natl Acad Sci U S A. 2009;106(32):13469-13474; Lambiase A. et al., Graefes Arch Clin Exp Ophthalmol Albrecht Von Graefes Arch Klin Exp Ophthalmol. 1997;235(12):780-785), diabetic retinopathy (Hammes HP et al., Mol Med. 1995;1(5):527-534, Mantelli DF. et al., Eur J Ophthalmol Published Online May 2013;9) and retinal degeneration causing photoreceptor loss (Lenzi L., et al., Vision Res. 2005;45(12):1491-1500; Lambiase A. et al., Graefes Arch Clin Exp Ophthalmol. 1996;234(1):S96-S100).

There is also clinical evidence that the administration of NGF is effective in patients with glaucoma (Lambiase A. et al., Proc Natl Acad Sci U S A. 2009;106(32):13469-13474), optic pathway gliomas (Falsini B. et al., Neurorehabil Neural Repair. 2011;25(6):512-520; Falsini B. et al., Brain J Neurol. 2016;139(Pt 2):404-414), retinitis pigmentosa (Falsini B. et al., J Transl Med. 2016;14;229(3):746-753), Non-Arteritic Anterior Ischemic Optic Neuropathy (NAION) (Chen Kai et al., Chin J Optom Ophthalmol Vis Sci, 2020, 22(10): 781-785), macular hole (Zhang et al., BMC Ophthalmol 2019, 19(1): 130) and non-neovascular age-related macular degeneration (Lambiase A. et al., Ann Ist Super Sanita. 2009;45(4):439-442). The administration of NGF shows protective effects and mitigates the retinal and otpic nerve damages and degeneration induced by these conditions. The current most used methods of delivering drugs to the eye are topical administration in the form of eye drops or direct injection to internal structures of the eye, such as intravitreal or retrobulbar injection. Typically, repeated administrations are necessary to maintain therapeutically relevant concentrations of the drug at the site of action.

Topical administration is non-invasive; however, it faces the challenge of achieving therapeutic drug concentrations in the retina and optic nerve due rapid drug clearance from the ocular surface and the difficulty of penetrating the inner eye tissues, with only a small fraction of the administered dose reaching the posterior segment of the eye.

The administration of a therapeutic agent directly into the internal structures of the eye may be perceived as invasive, uncomfortable, or even painful by patients, thus limiting their compliace with therapy.

Furthermore, the above-mentioned intravitreal injection is associated with some risks, such as irritation, subconjunctival hemorrhage, increased intraocular pressure (IOP), cataract formation, occurrence of post-injection infections (endophthalmitis), corneal abrasions, iritis and iatrogenic damage to the retina and lens, with possible retinal detachment. The likelihood of these complications occurring increases as the number of intravitreal administrations performed on the same patient increases (Nguyen QD, Rodrigues EB, Farah ME, Mieler WF, Do DV (eds): Retinal Pharmacotherapeutics. Dev Ophthalmol. Basel, Karger, 2016, vol 55, pp 63-70; Michael S. Ramos, et al., Ophthalmology Retina, Volume 5, Issue 7, 2021, pp. 625-632).

Because of this, intravitreal injection is not indicated in some pathological settings, such as glaucoma. In fact, the risk for post-injection IOP evelation is higher for patients with glaucoma or ocular hypertension. Good et al. demonstrated that patients with pre-existing glaucoma treated with intravitreal injections experience higher rates of elevated IOP when compared to patients without pre-existing glaucoma. It is possible that eyes with an already compromised aqueous humour outlow system may be more prone to developing elevated IOP in this setting (Good et al., Br J Ophthalmol 2011 Aug;95(8):1111-4).

It is therefore felt the need to deliver therapeutically effective amounts of NGF to the eye using alternative routes of administration to the above-discussed routes, to ensure greater compliance with therapy by patients and to minimize the risks associated with administration.

### SUMMARY OF THE INVENTION

The present inventors have now surprisingly found that, when nerve growth factor (NGF) is administered intranasally, it reaches the retina and the optic nerve at therapeutically effective amounts. Thus, the intranasal administration of NGF can be used for the prevention or treatment of disorders of the retina and of the optic nerve.

Accordingly, a first object of the invention is nerve growth factor (NGF) for use in the prevention or treatment of a retinopathy or of an optic nerve disorder in a subject, wherein said nerve growth factor (NGF) is administered intranasally to the subject.

A further object of the invention is a pharmaceutical composition comprising nerve growth factor (NGF) and a pharmaceutically acceptable excipient or carrier, for use in the prevention or treatment of a retinopathy or of an optic nerve disorder in a subject, wherein said pharmaceutical composition is administered intranasally to the subject.

A further object of the invention is the use of nerve growth factor (NGF) in the manufacture of a medicament for the prevention or treatment of a retinopathy or of an optic nerve disorder in a subject, wherein said nerve growth factor (NGF) is administered intranasally to the subject.

A further object of the invention is a method of preventing or treating a retinopathy or an optic nerve disorder in a subject, comprising intranasally administering to the subject a prophylactically or a therapeutically effective amount of nerve growth factor (NGF).

### BRIEF DESCRIPTION OF THE FIGURES

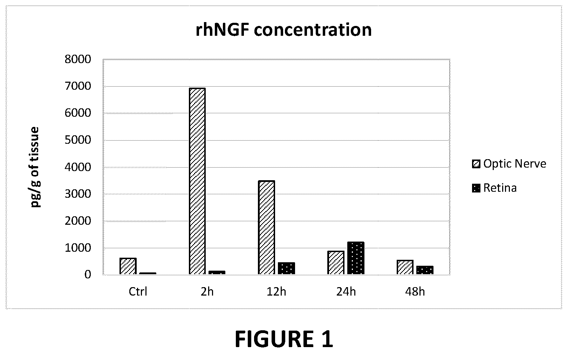

**Figure 1** shows the rhNGF concentration found in the retinas and in the optic nerves of mice treated in accordance with Example 1 below.

### DETAILED DESCRIPTION OF THE INVENTION

The present invention relates to nerve growth factor (NGF) for use in the prevention or treatment of a retinopathy or of an optic nerve disorder in a subject, wherein said nerve growth factor (NGF) is administered intranasally to the subject.

The term "prevention", as used herein, refers to the inhibition of onset of the disorder or to the delay in the onset of the disorder.

Thus, according to a preferred embodiment, said nerve growth factor (NGF) is for use in the inhibition of onset or in the delay in the onset of a retinopathy or of an optic nerve disorder in a subject.

The term "treatment", as used herein, refers to the eradication, the amelioration or the inhibition of the progression or slowing of the progression of the disorder being treated. Thus, according to a preferred embodiment, said nerve growth factor (NGF) is for use in the eradication, the amelioration, the inhibition of the progression or the slowing of the progression of a retinopathy or of an optic nerve disorder in a subject.

Preferably, the term "amelioration of the disorder being treated", as used herein, refers to the reduction of severity of the disorder being treated or of one or more of the symptoms associated thereof.

Thus, according to a preferred embodiment, said nerve growth factor (NGF) is for use in the reduction of the severity of a retinopathy or of an optic nerve disorder or of one of more of the symptoms associated thereof in a subject.

Preferably, said retinopathy is selected from the group consisting of diabetic retinopaty, retinis pigmentosa, retinal degeneration, retinal ischemia, phototoxic retinopathy, epiretinal membrane, macular hole, retinopathy of prematurity, retinal vascular occlusion, retinal detachment and age-related macular degeneration.

Preferably, said optic nerve disorder is selected from the group consisting of glaucoma, non-arteritic anterior ischemic optic neuropathy (NAION), optic pathway glioma, preferably optic pathway glioma-induced visual loss, and optic nerve atrophy. Preferably, said subject is a human subject.

Preferably, said nerve growth factor (NGF) for use according to the present invention is administered to the subject by daily administration for at least one week.

According to a preferred embodiment, said nerve growth factor (NGF) for use according to the present invention is administered to the subject by intermittent administration for at least 3 cycles, wherein each cycle comprises, preferably consists of, one week of daily administration with nerve growth factor (NGF) followed by two or three weeks of wash-out.

The term "wash-out" as used herein refers to a period in which nerve growth factor (NGF) is not administered to the subject.

According to another preferred embodiment, said nerve growth factor (NGF) for use according to the present invention is administered to the subject by daily administration for at least one month.

Preferably, said daily administration comprises from one to three intranasal administrations of nerve growth factor (NGF) per day.

More preferably, said daily administration comprises, preferably consists of, three intranasal administrations of nerve growth factor (NGF) per day.

Preferably, the amount of nerve growth factor (NGF) per each intranasal administration is between 5 µg and 720 µg, more preferably between 10 µg and 400 µg, even more preferably between 15 µg and 200 µg, most preferably about 60 µg.

The effective amount of said nerve growth factor (NGF) used in each administration, the duration of the treatment terapy, and the number of administrations per day are selected by the skilled person based on the characteristics and on the disorder of the subject to be treated, the severity of the symptoms and based on assessment tests carried out during the treatment.

Preferably, said nerve growth factor (NGF) for use according to the present invention is human nerve growth factor (hNGF).

Preferably, said human nerve growth factor (hNGF) has the aminoacid sequence of SEQ. ID NO.1 below

SEQ. ID NO.1:

SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVR

Alternatively, said human nerve growth factor (hNGF) has the aminoacid sequence of SEQ ID NO. 2 below:

SEQ ID NO. 2:
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVRRA

Alternatively, said human nerve growth factor (hNGF) is a mixture of nerve growth factors having aminoacid sequences of SEQ ID NO. 1 and SEQ ID NO. 2.

The human NGF of SEQ ID NO. 2 has an aminoacid sequence that only differ from the human NGF of SEQ ID NO. 1 for the presence of two additional amminoacids at the C-terminus. Both forms of human NGF are found in human cells and therefore are considered as wild type human NGF.

More preferably, said human nerve growth factor (hNGF) has the aminoacid sequence of SEQ. ID NO.1.

The human nerve growth factor having the aminoacid sequence of SEQ. ID. NO.1 is advantageous because it is is associated with an improved pharmacological profile in the context of the diseases of the invention. Indeed, as disclosed in WO2022167607A1, the human nerve growth factor having the aminoacid sequence of SEQ. ID. NO.1 predominantly activates TrKA-mediated pathways and inhibits apoptotic pathways mediated by p75^{NTR}, and thus is particularly useful in the treatment of pathologies where the effects of NGF on proliferation and survival are desired and where the proapoptotic effect of p75^{NTR} is detrimental.

Preferably, the nerve growth factor (NGF) for use according to the present invention is produced by recombinant DNA technology, preferably it is a human recombinant nerve growth factor (rhNGF).

Methods of producing rhNGF are known to the person skilled in the art, for example those described in WO0022119A1 and WO2013092776A1.

Preferably, the nerve growth factor (NGF) for use according to the present invention has a purity higher than 70%, more preferably higher than 80%, higher than 90%, higher than 95%, higher than 98% or higher than 99%. The purity of nerve growth factor (NGF) may be determined by conventional means known to those skilled in the art, preferably by HPLC analysis.

A further object of the present invention relates to a pharmaceutical composition comprising nerve growth factor (NGF) and at least one pharmaceutically acceptable excipient, for use in the prevention or treatment of a retinopathy or of an optic nerve disorder in a subject, wherein said pharmaceutical composition is administered intranasally to the subject.

Preferably, said nerve growth factor (NGF) is as described above.

Preferably, said retinopathy is selected from the group consisting of diabetic retinopathy, retinis pigmentosa, retinal degeneration, retinal ischemia, phototoxic retinopathy, epiretinal membrane, macular hole, retinopathy of prematurity, retinal vascular occlusion, retinal detachment and age-related macular degeneration.

Preferably, said optic nerve disorder is selected from the group consisting of glaucoma, non-arteritic anterior ischemic optic neuropathy (NAION), optic pathway glioma, preferably optic pathway glioma-induced vision loss, and optic nerve atrophy. Preferably, the pharmaceutical composition for use according to the invention is a liquid composition suitable for intranasal administration (liquid intranasal composition). Preferably, the pharmaceutical composition for use according to the present invention comprises nerve growth factor (NGF) and at least one pharmaceutically acceptable excipient suitable for intranasal administration.

Preferably, said at least one pharmaceutically acceptable excipient suitable for intranasal administration is selected from solvents, thickening agents, mucoadhesive agents, buffers, antioxidants, preservatives, and penetration enhancers.

Preferably, the pharmaceutical composition for use according to the present invention is administered to the subject by daily administration for at least one week.

According to a preferred embodiment, the pharmaceutical composition for use according to the invention is administered to the subject by intermittent administration for at least 3 cycles, wherein each cycle comprises, preferably consists of, one week of daily administration with the pharmaceutical composition followed by two or three weeks of wash-out.

The term "wash-out" as used herein refers to a period in which the pharmaceutical composition is not administered to the subject.

According to another preferred embodiment, the pharmaceutical composition for use according to the invention is administered to the subject by daily administration for at least one month.

Preferably, said daily administration comprises from one to three intranasal administrations of the pharmaceutical composition per day.

More preferably, said daily administration comprises, preferably consists of, three intranasal administrations of the pharmaceutical composition per day.

Preferably, the concentration of said nerve growth factor (NGF) in said liquid intranasal composition is between 20 µg/ml and 180 µg/ml , more preferably about 60 µg/ml.

The pharmaceutical composition for use according to the invention may be suitably formulated using appropriate methods known in the art or by the method disclosed in Remington's Pharmaceutical Science (recent edition), Mack Publishing Company, Easton Pa.

In a further aspect, the present invention relates to the use of nerve growth factor (NGF) in the manufacture of a medicament for the prevention or treatment of a rethinopathy or of an optic nerve disorder in a subject, wherein said nerve growth factor (NGF) is administered intranasally to the subject.

In a further aspect, the present invention relates to a method for the prevention or treatment of a retinopathy or of an optic nerve disorder in a subject, wherein the method comprises intranasally administering nerve growth factor (NGF) to the subject in a prophylactically or therapeutically effective amount.

A "prophylactically effective amount" according to the present invention means an amount sufficient to achieve prevention of the disease.

A "therapeutically effective amount" according to the present invention means an amount sufficient to achieve treatment of the disease.

Preferably, said nerve growth factor (NGF) is in the form of a pharmaceutical composition, as above described.

The invention will be further described in the following examples, which do not limit the scope of the invention described in the claims.

### EXPERIMENTAL SECTION

The present inventors have carried out experiments by administering NGF intranasally to different animal models. The concentration of NGF has been determined in the retina and in the optic nerve of the animals after intranasal administration to evaluate the feasibility of this route of administration to deliver NGF to these ocular structures in therapeutically effective amounts.

### Example 1

A biodistribution study of rhNGF in the optic nerve and in the retina at 2 hours, 12 hours, 24 hours and 48 hours post-instranasal administration in mice was carried out as follows.

### Animals

C57BL/6 male mice were used. Mice were housed in macrolon cages with filter hoods, in a room where the air was continuously filtered, thereby avoiding contamination. Animals arrived on site four days before rhNGF administration to allow optimal acclimation. During experiments, paired animals were caged at a constant temperature with a day/night cycle of 12/12 hours. Animals received water (control tap water) and nutrition ad libitum. Animal protocol was approved by the Animal Studies Committee of Languedoc Roussillon. This protocol and the procedures used comply with French legislation, which implements the European Directives (Reference Number: D3417223, APAFIS#23920-2020020320279696 v3). Two experimental sessions were carried out to cover the entire time course. To merge the obtained data, vehicle-treated animals of each experimental session, having a comparable ELISA response, were mediated. Mice were 10 weeks old and were randomly divided into 6 groups:
1) vehicle, which received only saline buffer; sampling at 2 h (n=3)
2) rhNGF-treated; sampling at 2 h (n=4)
3) vehicle, which received only saline buffer; sampling at 12h (n=3)
4) rhNGF-treated; sampling at 12h (n=4)
5) rhNGF-treated; sampling at 24h (n=4)
6) rhNGF- treated; sampling at 48h (n=4)

### Intranasal compound administration

rhNGF 2mg/ml was administered by intranasal route at 10 µl/animal. Using a dominant hand, the micropipette was leaded with 10 µl of compound or vehicle. The tip of the filled pipette was placed near the mouse's left nostril at 45-degree angle. The droplet was placed close enough to the mouse's nostril so that the mouse can inhale the droplet. Immediately after the mouse inhaled this small drop, the rest of the compound in the pipette tip was ejected to form another small droplet for the mouse to inhale through the same nostril about 2-3 sec later. After administration, the mouse was held in this position for 15 sec.

### Organ sampling

The temporal bone was opened, and the left optic nerves of all animals were sampled. Each sample was rapidly frozen using liquid nitrogen and stored at -80 °C. Then, the connective tissue in the orbital cavity surrounding the eye and any remaining tissue were cut to extract the intact eyeball. The retina was sampled using a scalpel and quickly frozen in liquid nitrogen and stored at -80 °C. Frozen tissues were cut into small parts, solubilized in 15 µl of sterile PBS completed with protease inhibitors (Fisher Scientific, France) and sonicated 3 times during 10 seconds on ice (Microson ultrasonic cell disruptorXL, Microsonic). Then, homogenates were centrifuged for 30 minutes at 10,000 rpm at 4°C, and 10 µl of supernatant stored at -20°C before rhNGF ELISA analysis.

### rhNGF analysis by ELISA

10 µl of diluted samples at 1/10 for the optic nerve and at 1/10 or 1/5 for retina were analyzed by ELISA method (Novus Biological, Ref. NBP2-62776). Standard references were diluted at 1000, 500, 250, 125, 62.5, 31.25, 15.63 and 0 pg/mL to perform the standard curve. The Standard working solution of different concentrations was added to the first two columns: each concentration of the solution was added into two wells side by side and the samples to other wells. The plate was covered with sealer provided in the kit and incubated for 90 min at 37°C. Then, the liquid of each well was removed and immediately 100 µL of Biotinylated Detection Ab working solution was added to each well. The plate was again covered with sealer provided in the kit and incubated for 1 hour at 37°C. After incubation, the solution from each well was aspirated and 350 µL of wash buffer was added and aspirated to each well. This wash step was repeated 3 times. 100 µL of HRP-conjugated working solution was added to each well. The plate was again covered with sealer provided in the kit and incubated for 30 min at 37°C. After washing, 90 µL of Substrate Reagent was added to each well and incubated for 15 min at 37°C. Finally, 50 µL of Stop Solution was added to each well and the optical density of each well was immediately determined using a microplate reader set to 450 nm.

### Results

The results are summarized in Table 1 below, which reports the mean rhNGF concentrations (pg/g of tissue) detected in the optic nerve and in the retina at the different time points analyzed, and in Figure 1.

**Table 1**

| | | **Time after treatment** | | | |
|---|---|---|---|---|---|
| **Tissue** | **Group** | 2 hours | 12 hours | 24 hours | 48 hours |
| Optic Nerve | Control (mean 2 and 12 h) | 615 | 615 | 615 | 615 |
| | Treated | 6923 | 3473 | 878 | 540 |
| Retina | Control (mean 2 and 12 h) | 55 | 55 | 55 | 55 |
| | Treated | 120 | 450 | 1210 | 305 |

A relevant increase of rhNGF concentration was observed in optic nerve of rhNGF treated group compared to the control group. 2 hours after single rhNGF administration by intranasal route, rhNGF was detected in optic nerve at a mean of 6923 pg/g of tissue. Progressive decrease of rhNGF concentration was observed at 12 hours, 24 hours and 48 hours post-treatment with a concentration of 3473 pg/g, 878 pg/g and 540 pg/g respectively. Taken together, these results demonstrate that rhNGF reaches the optic nerve when administered by intranasal route, with a peak concentration at 2 hours post treatment.

A slight increase of rhNGF concentration was observed in the retina of rhNGF-treated animals 2 hours post-administration compared to control animals. A relevant increase of rhNGF concentration was observed in the retina at 12 hours and 24 hours post-treatment in the rhNGF-treated group compared to the vehicle-treated group. At 12h post-administration, the compound was detected at 450 pg/g of tissue and at 24h post-administration the compound was detected at 1210 pg/g. The progressive increase of rhNGF concentration observed from 12h to 24h post-treatment reached a peak at 24h post-treatment. Then, decrease of rhNGF concentration was observed at 48h post-treatment, still remaining higher than the basal levels.

Based on in vitro data showing effective rhNGF concentration starting from approximately 10pM, the biodistribution results show that rhNGF administered intranasally in mice can reach therapeutically effective amounts at the timepoints of 2-24h in the optic nerve and of 12-48h in the retina.

### Example 2

A biodistribution study of rhNGF in the optic nerve and in the retina in New Zealand White Rabbits was carried out as follows.

New Zealand White rabbits (n=10) were treated either with control (physiological saline solution) or with rhNGF. The animals were divided into the following groups:
- Group 1 (control): animals treated with physiological saline solution, n=2;
- Group 2 (treatment): animals treated with rhNGF 0.6 mg/mL, n=4;
- Group 3 (treatment): animals treated with rhNGF 1.2 mg/mL, n=4.

The administration was carried out intranasally (0.5 mL/nostril) once a day, from day 1 to day 7. The animals were sacrificed 6±1 hours after the last administration on day 7.

Optic nerve and retina were homogenized before analysis: each optic nerve and retina samples was thawed in ice and 500 µL of Lysis buffer were added. Then, each sample was homogenized in ice by ultraturrax (3 cycles 30sec On/30sec Off at about 21000 g/min) and centrifuged for 15 minutes at 20000xg, 4°C. Supernatants were frozen at - 70°±10°C until analysis.

The results are shown in Table 2 below, which reports the rhNGF mean level (ng of rhNGF/g of tissue) detected in the optic nerve and in the retina of the analyzed animals.

**Table 2**

| **Tissue** | **Group** | **Animal n.** | **rhNGF level (ng of protein/g of tissue)** | **rhNGF mean level (ng of protein/g of tissue)** |
|---|---|---|---|---|
| **Optic Nerve** | 1 (Control) | 12 right | **0.0** | **1.9** |
| | | 12 left | **1.4** | |
| | | 22 right | **3.7** | |
| | | 22 left | **2.6** | |
| | 2 (rhNGF 0.6 mg/mL) | 15 right | **4.1** | **3.6** |
| | | 15 left | **2.1** | |
| | | 16 right | **1.8** | |
| | | 16 left | **0.0** | |
| | | 25 right | **4.1** | |
| | | 25 left | **6.0** | |
| | | 26 right | **2.9** | |
| | 3 (rhNGF 1.2 mg/mL) | 26 left | **7.5** | **6.9** |
| | | 19 right | **9.5** | |
| | | 19 left | **8.3** | |
| | | 20 right | **3.8** | |
| | | 20 left | **2.9** | |
| | | 29 right | **0.0** | |
| | | 29 left | **5.7** | |
| | | 30 right | **16.3** | |
| | | 30 left | **9.1** | |
| **Retina** | 1 (Control) | 12 right | **3.1** | **3.8** |
| | | 12 left | **4.0** | |
| | | 22 right | **1.4** | |
| | | 22 left | **6.6** | |
| | 2 (rhNGF 0.6 mg/mL) | 15 right | **6.2** | **4.9** |
| | | 15 left | **3.9** | |
| | | 16 right | **3.1** | |
| | | 16 left | **1.8** | |
| | | 25 right | **1.9** | |
| | | 25 left | **11.9** | |
| | | 26 right | **3.0** | |
| | | 26 left | **7.1** | |
| | 3 (rhNGF 1.2 mg/mL) | 19 right | **2.3** | **4.4** |
| | | 19 left | **1.7** | |
| | | 20 right | **3.2** | |
| | | 20 left | **4.5** | |
| | | 29 right | **4.6** | |
| | | 29 left | **2.2** | |
| | | 30 right | **6.5** | |
| | | 30 left | **9.9** | |

A relevant increase of rhNGF concentration was observed in optic nerve of rhNGF treated groups compared to the control group. The increase of rhNGF concentration in the optic nerve was dose-dependent: an almost double concentration of rhNGF was found in the optic nerve of group 3, which was treated with rhNGF 1.2 mg/mL, compared to group 2, which was treated with rhNGF 0.6 mg/mL (3.6 ng of rhNGF/g of tissue for group 2 vs. 6.9 ng of rhNGF/g of tissue for group 3).

A trend of increase of rhNGF concentration was found in the retina of rhNGF treated groups compared to the control group. This increase agrees with the data discussed in Example 1, where at the time immediately following administration there is only a slight trend of increase of rhNGF concentration in the retina, which then becomes relevant at longer times (12 h and 24 h).

Based on in vitro data showing effective rhNGF concentration starting from approximately 10pM, the biodistribution results show that rhNGF administered intranasally can reach therapeutically effective amounts in the optic nerve and also in the retina.

### Example 3

A biodistribution study of rhNGF in the optic nerve and in the retina in New Zealand White Rabbits was carried out as follows.

New Zealand White rabbits (n=11) were treated either with control (physiological saline solution) or rhNGF. The animals were divided into the following groups:
- Group 1 (control): animals treated with physiological saline solution, n=2;
- Group 2 (treatment): animals treated with rhNGF 1.2 mg/mL, n=6;
- Group 3 (treatment): animal treated with rhNGF 0.320 mg/mL, n=3.

A single administration was carried out intranasally (0.5 mL/nostril) at day 1. The animals were sacrificed 18±1 hours after the administration.

Optic nerve and retina samples were homogenized before analysis: each optic nerve and retina sample was thawed in ice and 500 µL of Lysis buffer were added. Then, each sample was homogenized in ice by ultraturrax (3 cycles 30sec On/30sec Off at about 21000g/min) and centrifuged for 15 minutes at 20000xg, 4°C. Supernatants were frozen at -70°±10°C until analysis.

The results are shown in Table 3 below, which reports the rhNGF mean levels detected in the optic nerve and in the retina of the analyzed animals.

**Table 3**

| **Tissue** | **Group** | **Animal n.** | **rhNGF level (ng of protein/ g of tissue)** | **rhNGF mean level (ng of protein/ g of tissue)** |
|---|---|---|---|---|
| **Optic Nerve** | 1 Control | 1 right | **24.2** | **31.3** |
| | | 1 left | **55.1** | |
| | | 2 right | **10.5** | |
| | | 2 left | **35.2** | |
| | 2 rhNGF 1.2 mg/mL | 3 right | **35.9** | **39.5** |
| | | 3 left | **10.9** | |
| | | 4 right | **52.3** | |
| | | 4 left | **33.2** | |
| | | 5 right | **48.0** | |
| | | 5 left | **71.5** | |
| | | 6 right | **46.9** | |
| | | 6 left | **30.1** | |
| | | 7 right | **50.0** | |
| | | 7 left | **26.3** | |
| | | 8 right | **28.4** | |
| | | 8 left | **40.6** | |
| | 3 rhNGF 0.320 mg/mL | 9 right | **35.7** | **32.7** |
| | | 9 left | **25.8** | |
| | | 10 right | **32.9** | |
| | | 10 left | **30.8** | |
| | | 11 right | **30.1** | |
| | | 11 left | **41.0** | |
| **Retina** | 1 Control | 1 right | **21.0** | **15.5** |
| | | 1 left | **20.6** | |
| | | 2 right | **8.3** | |
| | | 2 left | **12.0** | |
| | 2 rhNGF 1.2 mg/mL | 3 right | **8.2** | **17.0** |
| | | 3 left | **7.1** | |
| | | 4 right | **12.7** | |
| | | 4 left | **18.6** | |
| | | 5 right | **16.1** | |
| | | 5 left | **14.9** | |
| | | 6 right | **14.8** | |
| | | 6 left | **12.4** | |
| | | 7 right | **19.8** | |
| | | 7 left | **42.6** | |
| | | 8 right | **28.2** | |
| | | 8 left | **8.9** | |
| | 3 rhNGF 0.320 mg/mL | 9 right | **22.8** | **29.1** |
| | | 9 left | **14.1** | |
| | | 10 right | **52.5** | |
| | | 10 left | **17.4** | |
| | | 11 right | **41.1** | |
| | | 11 left | **26.5** | |

Although some groups showed a trend of increase of rhNGF levels in the optic nerve of the treated animals compared to control (group 2), this increase was of limited entity. Since the evaluation was carried out 18 hours after administration, these data are in line with the results obtained in Example 1, where an increase in optic nerve protein concentration is observed immediately after administration (peak at 2 hours) and then a steady decrease is observed at subsequent time points. In light of the slight increase found in treated group 2 compared to control, it is reasonable to assume that the concentration of the protein in the optic nerve increased in the first few hours after administration and then went through a decrease, which has not yet been completely exhausted for group 2, where a slightly higher level of the protein is observed.

An increase of rhNGF levels in the retina was observed particularly in group 3, where the level of rhNGF detected almost doubled the levels detected in the control group. This is surprising, considering that group 3 was treated with a a significantly lower rhNGF concentration than the other groups.

Based on in vitro data showing effective rhNGF concentration starting from approximately 10pM, these data thus confirm that, after intranasal administration, rhNGF is able to reach the retina and optic nerve in a therapeutically effective concentration and that the levels of rhNGF in the retina rise at later time points than in the optic nerve.

### Example 4

A preliminary biodistribution study of rhNGF in the optic nerve and in the retina in adult dogs was carried out as follows.

Three adult (5 to 7 months) Beagle male dogs (animal supplier: Marshall Bioresources) were used in the study.

One dog (control) was untreated.

The other two dogs (treatment 1 and treatment 2) were treated with rhNGF at the concentration of 1.2 mg/mL.

The administration of the liquid formulation was carried out intranasally (0.4 mL/nostril) via MAD (Mucosal Atomization Device).

Two administrations in each nostril were given 1 hour apart, and the dogs were sacrificed 30 minutes after the second administration. The total amount of rhNGF administered was 1.92 mg.

Optic nerve and retina were homogenized before analysis: each optic nerve and retina sample were thawed in ice and 500 µL of Lysis buffer were added. Then, each sample was homogenized in ice by ultraturrax (3 cycles 30sec On/30sec Off at about 21000 g/min) and centrifuged for 15 minutes at 20000xg, 4°C. Supernatants were frozen at - 70°±10°C until analysis.

The results are shown in Table 4 below, which reports the rhNGF mean levels detected in the optic nerve and in the retina of the analyzed animals.

**Table 4**

| **Tissue** | **Group** | **Animal** | **rhNGF level (pg of protein/g of tissue)** | **Mean rhNGF level (pg of protein/g of tissue)** |
|---|---|---|---|---|
| Retina | Control | right | 266.4 | 227.7 |
| | | left | 188.9 | |
| | rhNGF 1.2 mg/mL | 1 right | 268.2 | 332.8 |
| | | 1 left | 273.0 | |
| | | 2 right | 436.0 | |
| | | 2 left | 353.8 | |
| Optic Nerve | Control | right | 118.3 | 148.0 |
| | | left | 177.7 | |
| | rhNGF 1.2 mg/mL | 1 right | 181.4 | 218.2 |
| | | 1 left | 285.1 | |
| | | 2 right | 188.6 | |
| | | 2 left | 217.8 | |

As can be seen, a slight increase of rhNGF level was found in the retinas and in the optic nerves. Even if the study was performed with a limited number of animals, it indicates that the rhNGF intranasally delivered by means of MAD device is absorbed and distributes in the back of the eye tissues.

## Claims

1. Nerve growth factor for use in the prevention or treatment of a retinopathy or of an optic nerve disorder in a subject, wherein said nerve growth factor is administered intranasally to the subject.

2. Nerve growth factor for use according to claim 1, wherein said nerve growth factor is for use in the inhibition of onset or in the delay in the onset of a retinopathy or of an optic nerve disorder in said subject.

3. Nerve growth factor for use according to claim 1, wherein said nerve growth factor is for use in the eradication, the amelioration, the inhibition of the progression or the slowing of the progression of a retinopathy or of an optic nerve disorder in said subject.

4. Nerve growth factor for use according to any one of the preceding claims, wherein said retinopathy is selected from the group consisting of diabetic retinopaty, retinis pigmentosa, retinal degeneration, retinal ischemia, phototoxic retinopathy, epiretinal membrane, macular hole, retinopathy of prematurity, retinal vascular occlusion, retinal detachment and age-related macular degeneration.

5. Nerve growth factor for use according to any one of claims 1 to 4, wherein said optic nerve disorder is selected from the group consisting of glaucoma, non-arteritic anterior ischemic optic neuropathy (NAION), optic pathway glioma, preferably optic pathway glioma-induced visual loss, and optic nerve atrophy.

6. Nerve growth factor for use according to any one of the preceding claims, wherein said nerve growth factor is administered to the subject by daily administration for at least one week.

7. Nerve growth factor for use according to any one of the preceding claims, wherein said nerve growth factor is administered to the subject by intermittent administration for at least 3 cycles, wherein each cycle comprises one week of daily administration with nerve growth factor followed by two or three weeks of wash-out.

8. Nerve growth factor for use according to any one of claims 1 to 6, wherein said nerve growth factor is administered to the subject by daily administration for at least one month.

9. Nerve growth factor for use according to any one of claims 6 to 8, wherein said daily administration comprises from one to three intranasal administrations of nerve growth factors per day, preferably three intranasal administrations of nerve growth factors per day.

10. Nerve growth factor for use according to any one of the preceding claims, wherein the amount of nerve growth factor per each intranasal administration is between 5 µg and 720 µg, more preferably between 10 µg and 400 µg, even more preferably between 15 µg and 200 µg, most preferably about 60 µg.

11. Nerve growth factor for use according to any one of the preceding claims, wherein said nerve growth factor is human nerve growth factor, preferably recombinant human nerve growth factor.

12. Nerve growth factor for use according to any one of the preceding claims, wherein said nerve growth factor has the aminoacid sequence of SEQ. ID NO.1: 
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVR,
 or wherein said nerve growth factor has the aminoacid sequence of SEQ ID NO. 2: 
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVRRA,
 or wherein said nerve growth factor is a mixture of nerve growth factors having aminoacid sequences of SEQ ID NO. 1 and SEQ ID NO. 2.

13. A pharmaceutical composition comprising nerve growth factor and at least one pharmaceutically acceptable excipient, for use in the prevention or treatment of a retinopathy or of an optic nerve disorder in a subject, wherein said pharmaceutical composition is administered intranasally to the subject.

14. The pharmaceutical composition for use according to claim 13, wherein said pharmaceutical composition is a liquid composition suitable for intranasal administration.

15. The pharmaceutical composition for use according to claim 14, wherein the concentration of said nerve growth factor in the liquid intranasal composition is between 20 µg/ml and 180 µg/ml, more preferably about 60 µg/ml.
